# EUROPÄISCHE PATENTSCHRIFT

(11) **EP 1 649 045 B1**
(45) Veröffentlichungstag und Bekanntmachung des Hinweises auf die Patenterteilung: **25.06.2008**
(21) Anmeldenummer: 03813913.5
(22) Anmeldetag: 23.12.2003
(51) Int. Cl.: G01N 33/53, B01J 19/00

(54) **EINBAU VON HAPTEN-GRUPPEN BEI DER HERSTELLUNG VON TRÄGERN FÜR DIE ANALYTBESTIMMUNG**
INCORPORATION OF HAPTEN GROUPS DURING THE PRODUCTION OF CARRIERS FOR THE DETERMINATION OF ANALYTES
INCORPORATION DE GROUPES HAPTENE LORS DE LA PRODUCTION DE SUPPORTS POUR LA DETERMINATION DE SUBSTANCES A ANALYSER

(30) Priorität: 23.12.2002 DE 10260591; 23.12.2002 DE 10260592; 07.05.2003 DE 10320339
(43) Veröffentlichungstag der Anmeldung: 26.04.2006
(73) Patentinhaber: febit biotech GmbH, 69120 Heidelberg (DE)
(72) Erfinder: BEIER, Markus, 69121 Heidelberg (DE)
(74) Vertreter: Weiss, Wolfgang
(86) Internationale Anmeldenummer: PCT/EP2003/014820
(87) Internationale Veröffentlichungsnummer: WO 2004/059003

(56) Entgegenhaltungen:
- WO-A-00/13018
- WO-A-02/32567
- WO-A-02/089971
- US-A- 5 616 467

## Beschreibung

Die Erfindung betrifft ein Verfahren zur Herstellung eines Trägers, insbesondere eines mikrofluidischen Trägers, für die Bestimmung von Analyten.

In den letzten Jahren wurde mit der Technologie von auf einem Träger immobilisierten Rezeptorarrays, z.B. DNA-Chips, ein wertvolles Mittel geschaffen, das die schnelle und hochparallele Durchführung komplexer Analytbestimmungen erlaubt. Das den Rezeptorarrays zugrunde liegende biophysikalische Prinzip ist das der Wechselwirkung eines spezifischen immobilisierten Rezeptors mit einem in einer Flüssigphase vorhandenen Analyten, beispielsweise durch Nukleinsäurehybridisierung, wobei auf verschiedenen Bereichen des Trägers eine Vielzahl von Rezeptoren, z.B. Hybridisierungssonden, angebracht sind, die jeweils mit verschiedenen in der Probe vorhandenen Analyten, z.B. komplementären Nukleinsäureanalyten, spezifisch binden.

Um mit Rezeptorarrays, z.B. mit DNA-Chips, komplexe biologische Fragestellungen, wie Genexpressionsstudien, Targetvalidierung, Sequenzierungen oder Resequenzierungen bearbeiten zu können, ist eine effiziente Herstellung von Rezeptorarrays in hoher Qualität von grundlegender Bedeutung. Hierzu kann neben der Spotting-Technologie (Cheung et al., Nature Genet. Suppl. 1999, Vol. 21, 15-19) die Herstellung von DNA-Arrays auch in situ unter Verwendung von Phosphoramidit-Synthesebausteinen (Caruthers et al., Tetrahedron Lett., 1981, 1859) erfolgen. Hierbei lässt sich zwischen nasschemischen Verfahren (Maskos et al., Nucleic Acids Res. 1992, Vol. 20, 1679-1984) und photochemischen Verfahren (Pease, Proc. Natl. Acad. Sci., 1994, Vol. 91, 5022-5026) unterscheiden.

Ein Träger und ein Verfahren zur Analytbestimmung, die eine integrierte Rezeptorsynthese und Analyse erlauben, sind z.B. in WO 00/13018 beschrieben. Dort erfolgt die Rezeptorsynthese vorzugsweise unter Verwendung von photoaktivierbaren Rezeptorbausteinen. Alternativ wird eine Synthese der Rezeptorbausteine auch durch nasschemische Methoden offenbart. DE 101 22 357.9 und WO 02/089971 beschreiben ein Verfahren zur Rezeptorsynthese, das die Verwendung einer Kombination von photochemischen und nasschemischen Schritten beinhaltet.

Eine Aufgabe der vorliegenden Erfindung war es, ein Verfahren zur Herstellung eines Trägers für die Analytbestimmung bereitzustellen, das eine effiziente Kontrolle der Synthese von Rezeptoren auf dem Träger ermöglicht.

Die Aufgabe wird durch ein Verfahren gelöst, bei dem Hapten-Gruppen vor dem Syntheseprozess oder/und während eines oder mehrerer Schritte des Syntheseprozesses von Rezeptoren auf den Träger aufgebracht werden.

Ein erster Aspekt der Erfindung ist ein Verfahren zur Herstellung eines Trägers für die Bestimmung von Analyten, umfassend die Schritte:
(a) Bereitstellen eines Trägers,
(b) Leiten von Flüssigkeit mit Bausteinen für die Synthese polymerer Rezeptoren ausgewählt aus Nukleinsäuren und/oder Nukleinsäureanaloga, über den Träger,
(c) orts- oder/und zeitspezifisches Immobilisieren der Rezeptorbausteine an jeweils vorbestimmten Bereichen auf dem Träger und
(d) Wiederholen der Schritte (b) und (c) bis die gewünschten Rezeptoren
   an den jeweils vorbestimmten Bereichen synthetisiert worden sind, dadurch gekennzeichnet, dass vor, während oder/und nach der Synthese der Rezeptoren Hapten-Gruppen auf den Träger aufgebracht werden.

Ein zweiter Aspekt der Erfindung ist ein Verfahren zur Qualitätskontrolle von Rezeptorsynthesen auf einem Träger, umfassend die Schritte:
(a) Bereitstellen eines Trägers,
(b) flächiges Aufbringen von Haptengruppen auf die Trägeroberfläche,
(c) Durchführen einer Rezeptorsynthese auf dem Träger,
(d) Inkontaktbringen mit einem Hapten-Nachweisreagenz, das eine Detektion von Haptengruppen erlaubt,
(e) Auswerten der Haptengruppen-Detektion auf dem Träger und
(f) Korrelieren des Ergebnisses der Auswertung mit der Qualität oder/und Effizienz der Rezeptorsynthese, worin die zu synthetisierenden Rezeptoren als Nukleinsäuren und/oder Nukleinsäureanaloga ausgewählt werden.

Ein dritter Aspekt der Erfindung ist ein Verfahren zur Qualitätskontrolle von Rezeptorsynthesen, umfassend die Schritte:
(a) Bereitstellen eines Trägers,
(b) Durchführen einer Rezeptorsynthese auf dem Träger, wobei Haptengruppen während der Synthese in die Rezeptormoleküle an vorbestimmten Positionen eingebaut werden,
(c) Inkontaktbringen mit einem Hapten-Nachweisreagenz, das eine Detektion von Haptengruppen erlaubt,
(d) Auswerten der Haptengruppen-Detektion auf dem Träger und
(e) Korrelieren des Ergebnisses der Auswertung mit der Qualität oder/und Effizienz der Rezeptorsynthese, worin die zu synthetisierenden Rezeptoren als Nukleinsäure und/oder Nukleinsäureanaloga ausgewählt werden.

Die vorliegende Erfindung zeichnet sich insbesondere dadurch aus, dass das Verfahren zur Herstellung des Trägers mit einem Detektionssystem zur Analytbestimmung integriert werden kann. Dieses Detektionssystem kann zur integrierten Synthese und Analyse eingesetzt werden, insbesondere zum Aufbau komplexer Träger, z.B. Biochips, und zur Analyse komplexer Proben, z.B. zur Genom-, Genexpressions- oder Proteomanalyse.

Die Synthese der Rezeptoren erfolgt in situ auf dem Träger, beispielsweise indem Fluid mit Rezeptorsynthesebausteinen über den Träger geleitet wird, die Bausteine an den jeweils vorbestimmten Bereichen auf dem Träger ortsoder/und zeitspezifisch immobilisiert werden und diese Schritte wiederholt werden, bis die gewünschten Rezeptoren an den jeweils vorbestimmten Bereichen auf dem Träger synthetisiert worden sind.

Ein wesentliches Merkmal des erfindungsgemäßen Verfahrens besteht darin, dass Hapten-Gruppen flächig oder/und ortsaufgelöst, vor, während oder/und am Ende eines - vor dem eigentlichen Rezeptoraufbau erfolgenden - Spaceraufbaus oder/und vor, während oder/und am Ende der Rezeptorsynthese auf den Träger aufgebracht werden.

Der durch das erfindungsgemäße Verfahren hergestellte Träger ist vorzugsweise in einer Vorrichtung zur Bestimmung von Analyten integriert, umfassend
(i) eine Lichtquellenmatrix, vorzugweise eine programmierbare Lichtquellenmatrix, z.B. ausgewählt aus einer Lichtventilmatrix, einem Spiegelarray, einem UV-Laserarray und einem LED-Array,
(ii) einen Träger, vorzugweise einen mikrofluidischen Träger mit Kanälen, insbesondere mit geschlossenen Kanälen, in denen sich die vorbestimmten Bereiche mit den jeweils unterschiedlich immobilisierten Rezeptoren befinden, wobei die Kanäle vorzugsweise im Bereich von 10 µm bis 10000 µm, besonders bevorzugt im Bereich von 50 bis 250 µm, liegen und grundsätzlich in beliebiger Form ausgestaltet sein können, z.B. mit rundem, ovalem, quadratischem oder rechteckigem Querschnitt,
(iii) Mittel zur Zufuhr von Fluid zum Träger und zur Ableitung von Fluid aus dem Träger und
(iv) eine Detektionsmatrix, z.B. eine optische Detektionsmatrix, wie etwa eine CCD-Matrix oder/und eine elektronische Detektionsmatrix, wie sie in WO 00/13018 beschrieben ist.

In einer bevorzugten Ausführungsform bietet der Träger durch eine Einteilung in fluidische Subräume, die getrennt voneinander adressiert werden können, die Möglichkeit, die ortsspezifische Immobilisierung zu bestimmen. Ein Träger, der dieses Kriterium erfüllt, ist in WO 00/13018 beschrieben. Der Träger bietet dabei die Aufteilung der reaktiven Bereiche in 2 oder mehr Subräume.

Die Rezeptoren werden vorzugsweise ausgewählt aus Biopolymeren, die in situ auf dem Träger aus den entsprechenden Synthesebausteinen durch eine Kombination lichtgesteuerter und nasschemischer Prozesse sythetisiert werden können. Als Synthesebausteine können dabei sowohl monomere, z.B. Mononukleotide, als auch oligomere Bausteine, z.B. Di-, Tri- oder Tetranukleotide, etc., eingesetzt werden. Bevorzugt werden die Rezeptoren ausgewählt aus Nukleinsäuren wie DNA, RNA, Nukleinsäureanaloga wie Peptidnukleinsäuren (PNA). Besonders bevorzugt werden die Rezeptoren aus Nukleinsäuren und Nukleinsäureanaloga ausgewählt und in einem Nachweisverfahren zur Hybridisierung von komplementären Nukleinsäureanalyten eingesetzt. Die Immobilisierung der Rezeptoren auf der Oberfläche erfolgt vorzugsweise über Spacergruppen. Auch Spacergruppen können schrittweise aus entsprechenden Synthesebausteinen aufgebaut werden.

Die Spacer- bzw. Rezeptorsynthese umfasst vorzugsweise die Verwendung von Synthesebausteinen mit nasschemischen Schutzgruppen oder/und von Rezeptorbausteinen mit photochemischen Schutzgruppen. Gegebenenfalls können auch Synthesebausteine verwendet werden, die sowohl nasschemische als auch photochemische Schutzgruppen oder Hybridschutzgruppen, d.h. Gruppen, die zweistufig durch einen nasschemischen und eine photochemischen Schritt abspaltbar sind, tragen. Beispiele für nasschemische Schutzgruppen sind beliebige Schutzgruppen, wie aus dem Stand der Technik zur Synthese von Biopolymeren, wie etwa Nukleinsäuren oder Peptiden, auf festen Trägern bekannt sind. Bevorzugte Beispiele sind säurelabile Schutzgruppen, basenlabile Schutzgruppen, gegenüber Oxidation labile Schutzgruppen oder enzymatisch abspaltbare Schutzgruppen. Die Verwendung von säurelabilen Schutzgruppen, wie etwa Dimethoxytrityl, ist besonders bevorzugt. Für die photochemischen Syntheseschritte können beliebige photochemische Schutzgruppen, wie sie aus dem Stand der Technik zur Synthese von Biopolymeren, wie etwa Nukleinsäuren oder Peptiden, auf festen Trägern bekannt sind, eingesetzt werden. Bevorzugte Beispiele für photochemische Schutzgruppen sind in DE 101 05 079.8 und bevorzugte Beispiele für Hybridschutzgruppen sind in DE 101 05 077.1 beschrieben. Weiterhin können auch zweistufige Schutzgruppen verwendet werden, die einen photochemischen und einen nasschemischen Schritt zur Abspaltung bedürfen, wie in DE 101 32 025.6 beschrieben.

Das erfindungsgemäße Verfahren umfasst vorzugsweise die Herstellung eines Trägers mit mehreren, vorzugsweise mit mindestens 50 und besonders bevorzugt mit mindestens 100 verschiedenen Rezeptorbereichen, die mit jeweils unterschiedlichen Analyten in einer einzigen Probe reagieren können. Das erfindungsgemäße Verfahren kann zur Herstellung von Trägern eingesetzt werden, wobei die Rezeptoren in jedem Bereich des Trägers nur eine einzige Sequenz von Bausteinen enthalten. In einer weiteren Ausführungsform kann das erfindungsgemäße Verfahren jedoch auch zur Herstellung von Trägern eingesetzt werden, wobei die Rezeptoren in mindestens einem Bereich des Trägers mehrere verschiedene Sequenzen von Bausteinen enthalten.

Das erfindungsgemäße Verfahren beinhaltet das Aufbringen von Hapten-Gruppen auf den zur Herstellung von Rezeptoren verwendeten Träger. Diese Hapten-Gruppen werden vorzugsweise ausgewählt aus organischen Molekülen mit einem Molekulargewicht bis zu 2.000, insbesondere bis zu 1.000, die von einem spezifischen Bindepartner, z.B. einem Protein, wie etwa einem Antikörper, Streptavidin, Avidin oder einem Lektin, durch eine hochaffine Wechselwirkung erkannt werden. Die Bezeichnung "hochaffine Wechselwirkung" bedeutet in diesem Zusammenhang, dass die Wechselwirkung zwischen Haptengruppe und Bindepartner ausreichend stark ist, um eine Kontrolle des Einbaus von Hapten-Gruppen auf dem Träger unter jeweiligen Betriebsbedingungen mit einem entsprechenden Nachweisreagenz zu ermöglichen. Bevorzugte Beispiele für Haptene sind Digoxin und Digoxigenin sowie Dinitrophenol (DNP), die durch entsprechende Antikörper erkannt werden, bzw. Biotin oder Biotinanaloga, wie etwa Iminobiotin, Aminobiotin oder Desthiobiotin, die durch Streptavidin und Avidin erkannt werden.

Die Hapten-Gruppen können flächig oder ortsspezifisch auf den Träger aufgebracht werden. Auch Kombinationen von flächigem Aufbringen und ortsspezifischem Aufbringen, beispielsweise unter Verwendung von zwei oder mehreren unterschiedlichen Hapten-Gruppen, sind möglich. In diesem Zusammenhang bedeutet "flächiges Aufbringen", dass Hapten-Gruppen auf die Gesamtoberfläche des Trägers oder einen Teil davon aufgebracht werden, der Bereiche für die Rezeptorsynthese und benachbarte Bereiche, auf denen keine Rezeptorsynthese erfolgen soll, beinhaltet. Andererseits bedeutet "ortsspezifisches Aufbringen" dass die Hapten-Gruppen auf jeweils einzelne Bereiche oder Gruppen von Bereichen für die Rezeptorsynthese selektiv aufgebracht werden.

In einer bevorzugten Ausführungsform werden die Hapten-Gruppen in einen Spacer eingeführt. Ein solcher Spacer ist zwischen der eigentlichen Trägeroberfläche und dem Rezeptor angeordnet und kann dazu dienen, den Abstand des Rezeptors von der Oberfläche bzw. die Rezeptordichte innerhalb der vorbestimmten Bereiche auf einen optimalen Wert einzustellen. Die Synthese der Spacer erfolgt vorzugsweise ebenfalls durch orts- oder/und zeitspezifisches Immobilisieren einzelner Spacerbausteine, bis die gewünschten Spacer an den jeweils vorbestimmten Bereichen synthetisiert worden sind. Die Chemie des Spaceraufbaus kann analog der Chemie der Rezeptorsynthese unter Verwendung analoger Bausteine, die jedoch keine Rezeptorelemente enthalten, erfolgen, z.B durch Verwendung von Phosphoramiditbausteinen ohne Nukleobasen.

Durch Einbau von Hapten-Gruppen in den Spacer kann die Derivatisierung des Träger oder/und die Oberflächendichte der Spacermoleküle bestimmt werden. So kann beispielsweise während des Spaceraufbaus eine Hapten-Gruppe in den Spacer eingebaut werden und während oder/und nach dem Spaceraufbau ein flächiges Anfärben der Trägeroberfläche durch den spezifischen Bindepartner der Hapten-Gruppe erfolgen. Das bei dieser Anfärbung erhaltene Signal erlaubt Rückschlüsse auf die Qualität der Trägerderivatisierung und ermöglicht eine Bestimmung, ob der Träger für eine spätere Rezeptorsynthese überhaupt verwendbar ist. Ein Vorteil dieser Ausführungsform besteht darin, dass kein Einbau der Hapten-Gruppe in den Rezeptor erfolgt und sich die Hapten-Gruppe weder bei der folgenden Rezeptorsynthese noch bei einer Analytbestimmung störend auswirken kann.

In noch einer weiteren Ausführungsform kann die Hapten-Gruppe flächig auf die gesamte Trägeroberfläche aufgebracht werden. Dann wird nach bekannten Methode eine Rezeptorsynthese gegebenenfalls zusammen mit einer Spacersyntherse durchgeführt und anschließend die Oberfläche des Trägers durch den Hapten-Bindepartner angefärbt. An Bereichen, an denen eine erfolgreiche Rezeptorsynthese erfolgt ist, kann keine Anfärbung durch den Bindepartner erfolgen. Der Grad des Fehlens einer Anfärbung korreliert mit der Rezeptordichte. Auch diese Vorgehensweise hat den Vorteil, dass die zur Analytbestimmung verwendeten Rezeptoren eine Hapten-Gruppe tragen und sich die Hapten-Gruppe nicht störend bei der nachfolgenden Analytbestimmung auswirken kann.

In noch einer weiteren Ausführungsform der vorliegenden Erfindung werden die Hapten-Gruppen an einer oder mehreren Positionen in die auf dem Träger synthetisierten Rezeptoren einführt, z.B am Anfang, in der Mitte oder am Ende des Rezeptors. Diese Vorgehensweise erlaubt eine Kontrolle der Effizienz der Rezeptorsynthese über die Anzahl der in einen Bereich eingeführten Hapten-Gruppen.

Die Einführung der Hapten-Gruppen kann reversibel oder irreversibel erfolgen. Ein reversibles Einführen der Hapten-Gruppen hat den Vorteil, dass die Gruppen zu definierten Zeitpunkten während bzw. nach der Rezeptorsynthese wieder abgespalten werden können und somit nicht zu einer Beeinträchtigung der Analytbindung an den Rezeptor führen können.

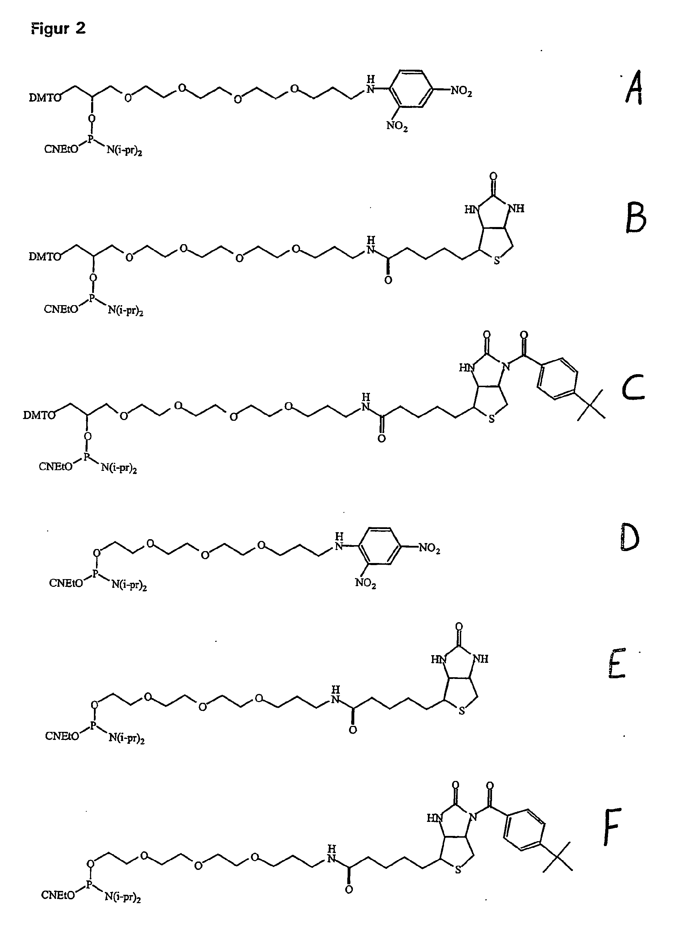

Die unterschiedlichen Arten der Einführung von Hapten-Gruppen ermöglichen ein reversibles und irreversibles Monitoring. Ein reversibles Monitoring bedeutet, dass nach Einbau von derartigen Reagenzien ein weiterer Kettenaufbau an dieser Position möglich ist, d.h. diese Reagenzien eignen sich nicht nur für eine terminale, sondern auch für eine interne Verwendung/Qualitätskontrolle der Rezeptorsynthese (Beispiel Figur 2A, B, C). Ein irreversibles Monitoring bedeutet, dass nach Einbau von derartigen Reagenzien kein weiterer Kettenaufbau an diese Position mehr möglich ist, d.h. diese Reagenzien eignen sich für eine terminale Verwendung/Qualitätskontrolle der Rezeptorsynthese (Beispiel Figur 2 D, E, F).

Weiterhin soll die vorliegenden Erfindung durch die nachfolgenden Figuren verdeutlicht werden:

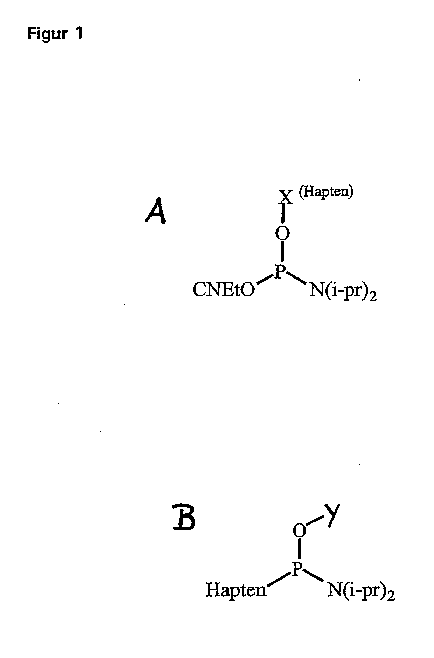

**Figur 1** zeigt zwei Ausführungsformen von Hapten-Gruppen-enthaltenden Synthonen, die für die Herstellung von Rezeptoren oder/und Spacern eingesetzt werden können. Es handelt sich dabei um Phosphoramidit-Synthone. Es können entsprechend jedoch auch andere Synthontypen eingesetzt werden.
**Figur 1A** zeigt einen Phosphoramiditbaustein, der mit einer Diisopropylaminogruppe, einer Cyanoethoxygruppe und einer weiteren Funktionalität X, die eine Hapten-Gruppe trägt, substituiert ist. Die Funktionalität X kann beispielsweise eine Nukleobase oder/und eine Spacer-Einheit beinhalten.
Gemäß **Figur 1B** ist ein Phosphoramiditbaustein gezeigt, der mit einer Diisopropylaminogruppe, einer gegebenenfalls über einen Spacer an das Phosphoratom gebundenen Hapten-Gruppe und einer Gruppe O-Y modifiziert ist, wobei Y eine Spacergruppe oder eine Nukleobase enthalten kann. Hier ist im Gegensatz zu Figur 1A die Hapten-Funktion direkt an die Phosphitamid-Einheit gebunden.
In **Figur 2** sind spezifische Ausführungsformen des in Figur 1A gezeigten Phosphoramiditderivats dargestellt. Bei den in **Figur 2A** und **D** gezeigten Derivaten ist die Hapten-Gruppe eine Dinitrophenylgruppe. Bei den in **Figur 2B** und **E** gezeigten Derivaten ist die Hapten-Gruppe eine Biotingruppe. Bei den in **Figur 2C** und **F** gezeigten Derivaten ist die Hapten-Gruppe eine Biotingruppen, die mit einer tert-Butylbenzoesäure-Gruppe geschützt ist. Die Figuren 2A-C zeigen Derivate, die zusätzlich eine DMT (Dimethoxytritylgruppe) tragen. Dies hat den Vorteil, dass nach Kopplung mit diesem Reagenz die Oligonukleotidsynthese nach Abstraktion der terminalen DMT-Gruppe fortgeführt werden kann. Diese Reagenzien eignen sich somit zusätzlich für eine interne Markierung eines Oligonukleotids mit Haptenen, während es sich bei Figur 2D-E um terminale Hapten-Markierungs-Reagenzien handelt.
Die in Figur 2A-C gezeigten Derivate sind für ein reversibles Monitoring geeignet, während die in Figur 2D-F gezeigten Derivate für ein irreversibles Monitoring eingesetzt werden können.

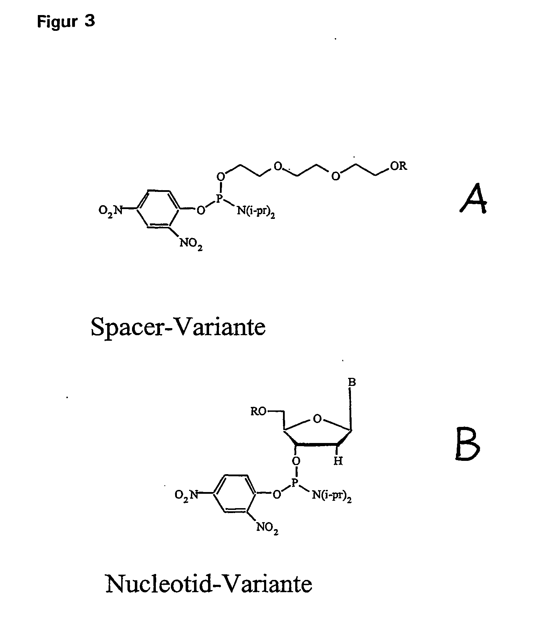

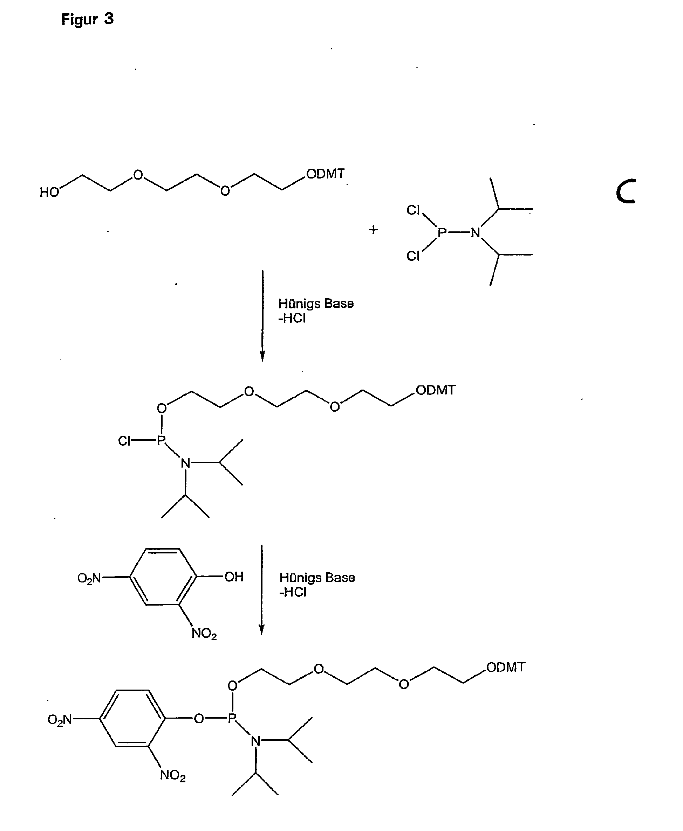

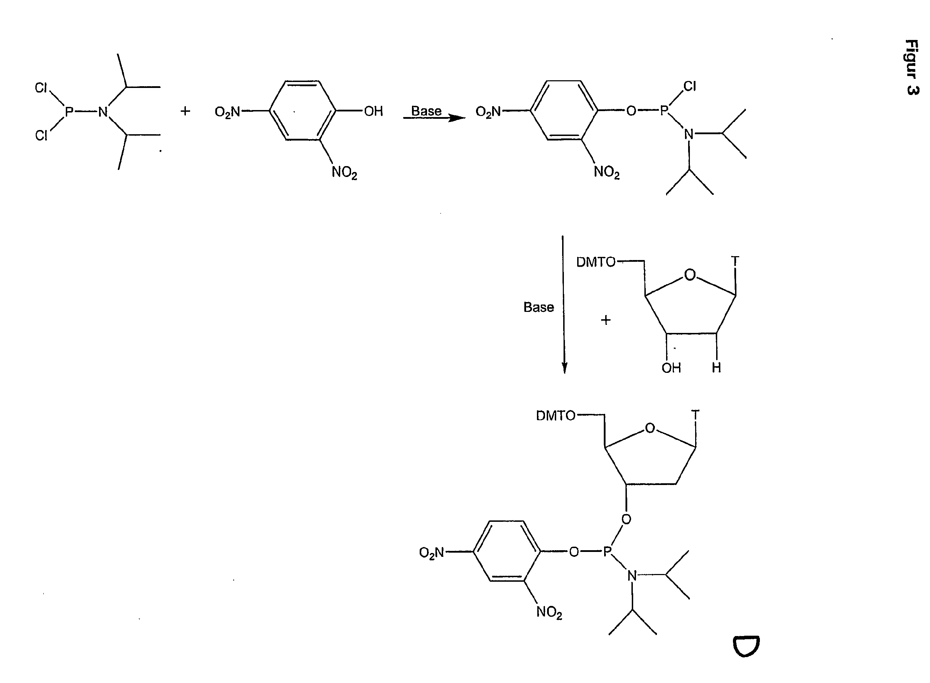

**Figur 3** zeigt spezifische Beispiele des in Figur 1B dargestellten Derivats. Als Hapten-Gruppe ist eine Dinitrophenylgruppe vorhanden. Die Gruppe Y ist bei der Variante gemäß **Figur 3A** eine Spacergruppe, wobei R eine Schutzgruppe, z.B. eine fotolabile oder säurelabile Schutzgruppe, darstellt. Bei der in **Figur 3B** gezeigten Variante ist Y eine Nukleosidgruppe umfassend eine Desoxyiboseeinheit und Nukleobase (B), wobei die 5'-Position der Desoxyriboseeinheit durch eine Schutzgruppe R blockiert ist. Beide Reagenzien eignen sich zusätzlich für eine interne Markierung eines Oligonukleotids mit Haptenen.
Die Herstellung der Verbindungen gemäß Figur 3A erfolgt entsprechend dem Syntheseschema in **Figur 3C**. Die Herstellung der Verbindung gemäß Figur 3B erfolgt entsprechend dem Syntheseschema in **Figur 3D**, wobei als Nukleobase z.B. T verwendet wird.

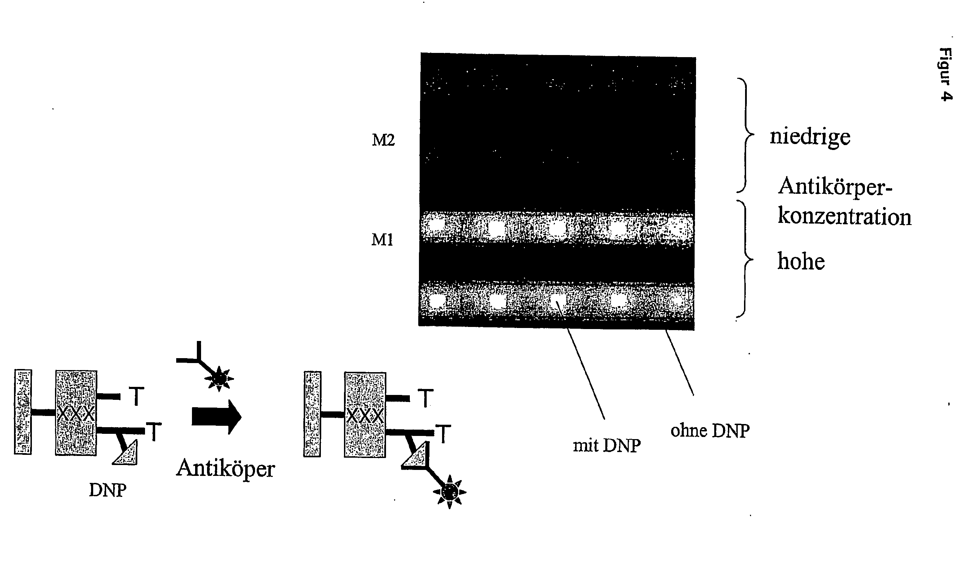

**Figur 4** zeigt einen ortsaufgelösten Einbau des Haptens DNP auf einem Träger an Bereiche für die Rezeptorsynthese und dessen Nachweis mit einem Anti-DNP-Antikörper (markiert mit dem Fluoreszenzfarbstoff Alexa 488).

Nach der flächigen Kopplung von zwei Spacereinheiten (X) wurde ortsaufgelöst eine DNP-Gruppe (DNP) aufgebracht. Nachfolgend wurde flächig eine Thymidineinheit (T) aufgebracht.

Die Detektion erfolgte mit einem anti-DNP-Antikörper, der mit Alexa-488 markiert war. Nur diejenigen Positionen ergeben ein Signal, auf die eine DNP-Einheit kondensiert wurde. Die Signalstärke ist abhängig von der Antikörperkonzentration.

Als DNP-Gruppe fand ein Baustein vom Typ Figur 2A Verwendung, d.h. es wurde ein reversibles Monitoring durchgeführt. Nach Abstraktion der an der DNP-Einheit befindlichen DMT-Gruppe konnte die Rezeptorsynthese fortgeführt werden.

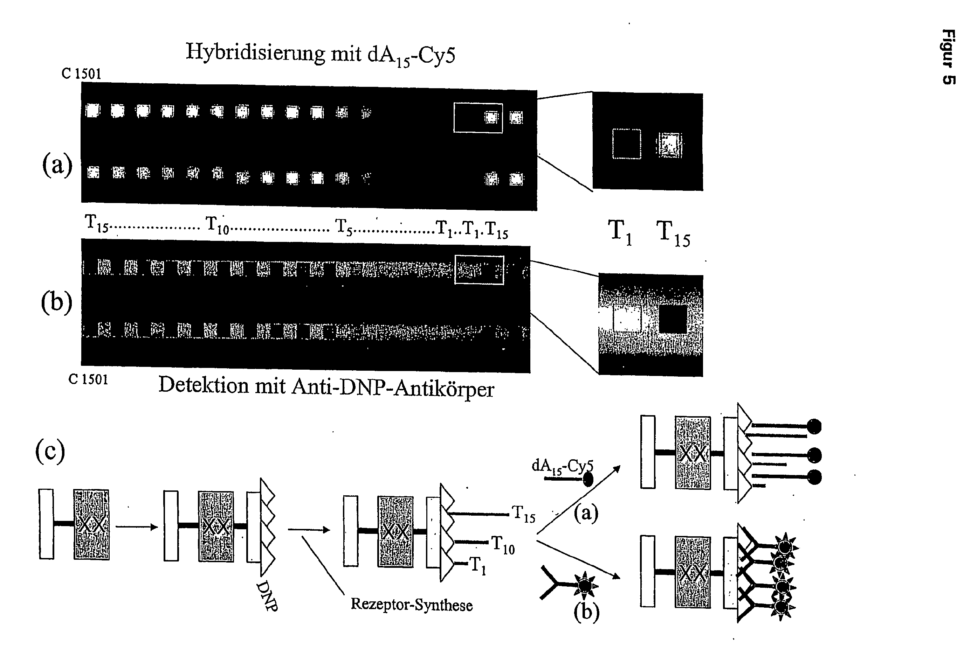

**Figur 5** zeigt die flächige Kopplung von zwei Spacer-Einheiten (X) und das abschließende flächige Aufbringen von DNP auf den Träger vor einer ortsaufgelösten Rezeptorsynthese von homomeren Thymidineinheiten.
(a) Es wurde eine Hybridisierung mit einer Cy5-markierten, komplementären dA15-Sequenz durchgeführt. Hier sind mit abnehmender Signalintensität die Hybridisierungen auf den komplementären Thymidinsequenzen (von T₁₅...T₁) zu erkennen.
(b) Es wurde eine Detektion mit einem anti-DNP-Antikörper durchgeführt, der mit Alexa-488 markiert ist.
   Hier ergibt sich ein flächiges Signal, das auf das Vorhandensein von DNP-Einheiten beruht, das nur an den Stellen, an denen Thymidineinheiten aufgebaut wurden, unterbrochen wird (Negativsignal). Zudem ist dieses Negativ-Signal umso stärker, je länger die Thymidinsequenz ist. Die Länge des Rezeptors, d.h. der Erfolg einer Synthese kann durch zunehmendes Negativ-Signal (abnehmendes Positivsignal) detektiert werden (negative Korrelation von Synthese und Detektionssignal).
   Als DNP-Gruppe fand ein Baustein des Typ gemäß Figur 2A Verwendung, d.h. es wurde ein reversibles Monitoring durchgeführt. Nach Abstraktion der an der DNP-Einheit befindlichen DMT-Gruppe konnte die Rezeptorsynthese fortgeführt werden.
(c) Es ist eine schematische Darstellung der Prozessorschritte gezeigt. Ausgangspunkt ist ein Träger mit zwei Spacer-Einheiten X; dann erfolgte eine flächige Kondensation mit DNP; dann erfolgt eine Rezeptorsynthese ortsaufgelöst (Sequenzen T₁ bis T₁₅); dann erfolgt entweder eine Detektion mit (a) Cy5-markierten dA15 oder (b) mit anti-DNP-Antikörpern.

Somit kann die Länge der aufgebauten Oligonukleotide (T₁-T₁₅) durch Reaktion mit den anti-DNP-Antikörpern ermittelt werden. Das heißt, dieses Verfahren eignet sich zur Qualitätskontrolle einer Rezeptorsynthese, da die Erkennung der Sondenlänge und damit auch die Effizienz der an diese Position erfolgten Synthese unabhängig von einer Sequenz mit einem Antikörper universell erfolgen kann. Statt einer Kontrollhybridisierung, die die Kenntnis der aufgebauten Rezeptorsequenzen voraussetzt, kann eine universelle Erkennung von beliebig vielen unterschiedlichen Sequenzen über den Antikörper erfolgen.

Der in Figur 5 gezeigte Träger mit Oligo-T-Rezeptoren kann ohne Weiteres für Hybridisierungsexperimente, beispielsweise mit fluoreszenzmarkierten dA₁₅-Sonden eingesetzt werden. Man erhält eine von der Länge der T-Sonde abhängige Signalintensität.

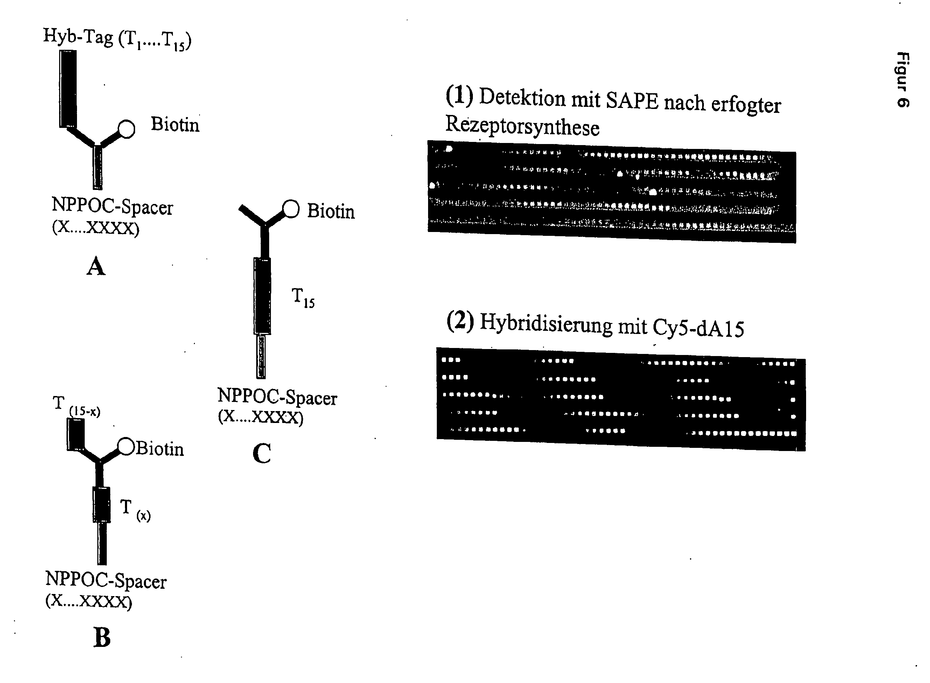

**Figur 6** zeigt den Einbau von Biotingruppen in einen Spacer. Nach der ortsaufgelösten Kopplung mit 1 bis 4 Spacer-Einheiten (X ... XXXX) wurde ortaufgelöst eine Biotin-Gruppe eingeführt, entweder:
(A) distal, d.h. vor Rezeptorsynthese,
(B) an beliebiger Position während der Rezeptorsynthese (hier zwischen T₁ und T₁₅)
(C) terminal, d.h. nach beendeter Rezeptorsynthese.

Es bestehen 3 Möglichkeiten der Detektion:
(1) Detektion mit einem Streptavidin-Phycoerythrin-Konjugat (SAPE) nach beendeter Rezeptorsynthese
   Hier kann die Länge/der Erfolg der ortsaufgelösten Synthese direkt an den ortsaufgelösten Signalintensitäten abgelesen werden. Die gefundenen Intensitäten verhalten sich längenabhängig, d.h. nach Anfärbung mit SAPE ergibt eine T₁₅-Sequenz ein höheres Signal als eine entsprechende kürzere Sequenz.
(2) Hybridisierung mit einer Cy5-markierten, komplementären dA15-Sequenz
   Hier sind mit abnehmender Signalintensität die Hybridisierungen auf den komplementären Thymidinsequenzen (von T₁₅ ... T₁) zu erkennen.

Durch Waschen, z.B. mit DMSO/NMI, Methanol, Ethanol, Acetonitril etc. kann das PE-Streptavidinkonjugat abgestrippt werden.

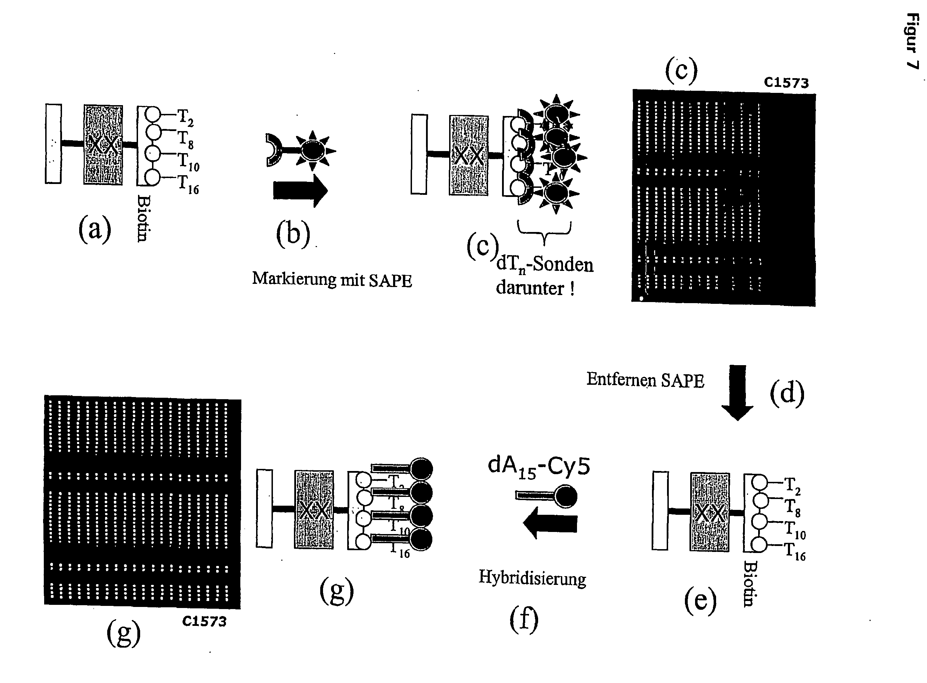

**Figur 7** zeigt die flächige Kopplung von zwei Spacer-Einheiten, das Aufbringen einer Biotin-Gruppe und eine anschließende Rezeptorsynthese.
(a) Nach der flächigen Kopplung mit zwei Spacereinheiten (X) wurde flächig eine Biotin-Gruppe aufgebracht. Nachfolgend wurde eine ortsaufgelöste Synthese von homomeren Thymidineinheiten (T₂ ... T₁₆) durchgeführt.
(b) Detektion mit einem SAPE-Konjugat
(c) Es werden ortsaufgelöst Signale an allen Positionen detektiert, an den bei der Rezeptorsynthese Thymidin-Sequenzen aufgebaut wurden. Es werden Positiv-Signale erzeugt. Die Intensitätsverteilung der Signale folgt der Dichte bzw. Länge der Rezeptormoleküle. Das heißt, bereits ohne Hybridisierung kann direkt nach der Synthese der Erfolg der Rezeptorsynthese überprüft werden.
(d) Es erfolgt die Entfernung der SAPE-Signalträgermoleküle. Hierzu wird der DNA-Chip vorzugsweise mit einem organischen Lösungsmittel gewaschen (z.B. Acetonitril, Ethanol, DMSO).
(e) Der Träger liegt in seinem ursprünglichen Zustand ohne SAPE-Signalträgermoleküle vor.
(f) Hybridisierung mit Cy5-markierter, komplementärer dA15-Sequenz.
(g) Hier sind mit abnehmender Signalintensität die Hybridisierungen auf den komplementären Thymidinsequenzen (von T₁₆ ... T₂) zu erkennen.

## Patentansprüche

1. Verfahren zur Herstellung eines Trägers für die Bestimmung von NukleinsäureAnalyten, umfassend die Schritte:
(a) Bereitstellen eines Trägers,
(b) Leiten von Flüssigkeit mit Bausteinen für die Synthese polymerer Rezeptoren, ausgewählt aus Nukleinsäuren und/oder Nukleinsäureanaloga, über den Träger,
(c) orts- oder/und zeitspezifisches Immobilisieren der Rezeptorbausteine an jeweils vorbestimmten Bereichen auf dem Träger und
(a) Wiederholen der Schritte (b) und (c) bis die gewünschten Rezeptoren an den jeweils vorbestimmten Bereichen synthetisiert worden sind,
**dadurch gekennzeichnet,**
**dass** vor, während oder/und nach der Synthese der Rezeptoren Hapten-Gruppen auf den Träger aufgebracht werden.

2. Verfahren zur Qualitätskontrolle von Rezeptorsynthesen auf einem Träger, umfassend die Schritte:
(a) Bereitstellen eines Trägers,
(b) flächiges Aufbringen von Haptengruppen auf die Trägeroberfläche,
(c) Durchführen einer Rezeptorsynthese auf dem Träger,
(d) Inkontaktbringen mit einem Hapten-Nachweisreagenz, das eine Detektion von Haptengruppen erlaubt,
(e) Auswerten der Haptengruppen-Detektion auf dem Träger und
(f) Korrelieren des Ergebnisses der Auswertung mit der Qualität oder/und Effizienz der Rezeptorsynthese, worin die zu synthetisierenden Rezeptoren aus Nukleinsäuren und/oder Nukleinsäureanaloga ausgewählt werden.

3. Verfahren zur Qualitätskontrolle von Rezeptorsynthesen, umfassend die Schritte:
(a) Bereitstellen eines Trägers,
(b) Durchführen einer Rezeptorsynthese auf dem Träger, wobei Haptengruppen während der Synthese in die Rezeptormoleküle an vorbestimmten Positionen eingebaut werden,
(c) Inkontaktbringen mit einem Hapten-Nachweisreagenz, das eine Detektion von Haptengruppen erlaubt,
(d) Auswerten der Haptengruppen-Detektion auf dem Träger und
(e) Korrelieren des Ergebnisses der Auswertung mit der Qualität oder/und Effizienz der Rezeptorsynthese, worin die Rezeptormoleküle aus Nukleinsäuren und/oder Nukleinsäureanaloga ausgewählt werden.

4. Verfahren nach einem der Ansprüche 1 bis 3,
**dadurch gekennzeichnet,**
**dass** man einen mikrofluidischen Träger mit Kanälen, vorzugsweise mit geschlossenen Kanälen verwendet, in denen vorbestimmte Bereiche mit immobilisierten Rezeptoren entstehen.

5. Verfahren nach einem der Ansprüche 1 bis 4,
**dadurch gekennzeichnet,**
**dass** man einen Träger mit mehreren, vorzugsweise mit mindestens 50 und besonders bevorzugt mit mindestens 100 verschiedenen Rezeptorbereichen herstellt.

6. Verfahren nach einem der Ansprüche 1 bis 5,
**dadurch gekennzeichnet,**
**dass** die Hapten-Gruppen ausgewählt werden aus organischen Molekülen mit einem Molekulargewicht bis zu 2.000, die von einem spezifischen Bindepartner durch eine hochaffine Wechselwirkung erkannt werden.

7. Verfahren nach Anspruch 6,
**dadurch gekennzeichnet,**
**dass** die Hapten-Gruppen ausgewählt werden aus Digoxin, Digoxigenin, Dinitrophenol und Biotin oder Biotinanaloga.

8. Verfahren nach einem der Ansprüche 1 bis 7,
**dadurch gekennzeichnet,**
**dass** ein flächiges Aufbringen der Hapten-Gruppen auf den Träger erfolgt.

9. Verfahren nach einem der Ansprüche 1 bis 8,
**dadurch gekennzeichnet,**
**dass** ein ortsspezifisches Aufbringen der Hapten-Gruppen auf den Träger erfolgt.

10. Verfahren nach einem der Ansprüche 1 bis 9,
**dadurch gekennzeichnet,**
**dass** die Hapten-Gruppen direkt auf die Oberfläche des Trägers aufgebracht werden.

11. Verfahren nach einem der Ansprüche 1 bis 10,
**dadurch gekennzeichnet,**
**dass** die Hapten-Gruppen in Spacermoleküle eingeführt werden, die zwischen der Trägeroberfläche und den Rezeptoren angeordnet sind.

12. Verfahren nach einem der Ansprüche 1 bis 11,
**dadurch gekennzeichnet,**
**dass** die Hapten-Gruppen an einer oder mehreren Positionen in die auf dem Träger synthetisierten Rezeptoren eingeführt werden.

13. Verfahren nach einem der Ansprüche 1 bis 12,
**dadurch gekennzeichnet,**
**dass** ein reversibles Aufbringen der Hapten-Gruppen erfolgt.

14. Verfahren nach einem der Ansprüche 1 bis 12,
**dadurch gekennzeichnet,**
**dass** ein irreversibles Aufbringen der Hapten-Gruppen erfolgt.

15. Verwendung von Hapten-Gruppen zur Kontrolle der Synthese von Rezeptoren auf einem Träger, worin die Rezeptoren aus Nukleinsäuren und/oder Nukleinsäureanaloga ausgewählt werden.

## Claims

1. Method for producing a carrier for the determination of nucleic acid analytes, including the steps:
(a) providing a carrier,
(b) passing liquid with building blocks for synthesizing polymeric receptors, selected from nucleic acids and/or nucleic acid analogues, over the carrier,
(c) site- or/and time-specifically immobilizing the receptor building blocks in each case on predetermined zones on the carrier and
(d) repeating steps (b) and (c) until the desired receptors have been synthesized in each case on the predetermined zones,
**characterized in that** hapten groups are applied to the carrier before, during or/and after the synthesis of the receptors.

2. Method for the quality control of receptor syntheses on a carrier, comprising the steps;
(a) providing a carrier,
(b) applying in planar fashion hapten groups to the carrier surface,
(c) carrying out a receptor synthesis on the carrier,
(d) contacting with a hapten detection reagent which permits detection of hapten groups,
(e) evaluating the hapten group detection on the carrier and
(f) correlating the result of the evaluation with the quality or/and efficiency of the receptor synthesis, wherein the receptors to be synthesized are selected from nucleic acids and/or nucleic acid analogues.

3. Method for the quality control of receptor syntheses, including the steps:
(a) providing a carrier,
(b) carrying out a receptor synthesis on the carrier, with hapten groups being incorporated during the synthesis into the receptor molecules at predetermined positions,
(c) contacting with a hapten detection reagent which permits detection of hapten groups,
(d) evaluating the hapten group detection on the carrier and
(e) correlating the result of the evaluation with the quality or/and efficiency of the receptor synthesis, wherein the receptor molecules are selected from nucleic acids and/or of nucleic acid analogues.

4. Method according to any of Claims 1 to 3, **characterized in that** a microfluidic carrier with channels, preferably with closed channels, in which predetermined zones with immobilized receptors are produced is used.

5. Method according to any of Claims 1 to 4, **characterized in that** a carrier is produced with a plurality of, preferably with at least 50 and particularly preferably with at least 100, different receptor zones.

6. Method according to any of Claims 1 to 5, **characterized in that** the hapten groups are selected from organic molecules having a molecular weight of up to 2,000, which are recognized by a specific binding partner through a high-affinity interaction.

7. Method according to Claim 6, **characterized in that** the hapten groups are selected from digoxin, digoxigenin, dinitrophenol and biotin or biotin analogs.

8. Method according to any of Claims 1 to 7, **characterized in that** the hapten groups are applied in a planar fashion to the carrier.

9. Method according to any of Claims 1 to 8, **characterized in that** the hapten groups are applied in a site-specific manner to the carrier.

10. The method according to any of Claims 1 to 9, **characterized in that** the hapten groups are applied directly to the surface of the carrier.

11. Method according to any of Claims 1 to 10, **characterized in that** the hapten groups are inserted into spacer molecules which are disposed between the carrier surface and the receptors.

12. Method according to any of Claims 1 to 11, **characterized in that** the hapten groups are inserted at one or more positions into the receptors synthesized on the carrier.

13. Method according to any of claims 1 to 12, **characterized in that** the hapten groups are applied reversibly.

14. Method according to any of Claims 1 to 12, **characterized in that** the hapten groups are applied irreversibly.

15. Use of hapten groups for controlling the synthesis of receptors on a carrier, wherein the receptors are selected from nucleic acids and/or nucleic acid analogues.

## Revendications

1. Procédé de préparation d'un support pour la détection d'acide nucléique, comprenant les étapes de :
(a) préparation d'un support,
(b) passage, sur le support, d'un liquide avec des éléments pour la synthèse de récepteurs polymères, choisis parmi les acides nucléiques et/ou des analogues d'acide nucléique,
(c) immobilisation, spécifique d'un site et/ou d'un temps, des éléments de récepteur sur des zones prédéterminées sur le support, et
(d) répétition des étapes (b) et (c) jusqu'à ce que les récepteurs souhaités aient été synthétisés sur les zones prédéterminées,
**caractérisé en ce qu'**avant, pendant et/ou après la synthèse des récepteurs, des groupes haptène sont appliqués sur le support.

2. Procédé pour le contrôle de qualité de la synthèse de récepteurs sur un support, comprenant les étapes de :
(a) préparation d'un support,
(b) application de groupes haptène sur la surface du support,
(c) réalisation de la synthèse des récepteurs sur le support,
(d) mise en contact avec un réactif de détection d'haptènes, qui permet une détection des groupes haptène,
(e) évaluation de la détection des groupes haptène sur le support, et
(f) corrélation des résultats de l'évaluation avec la qualité et/ou l'efficacité de la synthèse des récepteurs, où les récepteurs à synthétiser sont choisis parmi les acides nucléiques et/ou les analogues d'acide nucléique.

3. Procédé pour le contrôle de qualité de la synthèse de récepteurs, comprenant les étapes de :
(a) préparation d'un support,
(b) réalisation de la synthèse des récepteurs sur le support, des groupes haptène étant incorporés pendant la synthèse, dans la molécule de récepteur en des positions prédéterminées,
(c) mise en contact avec un réactif de détection d'haptènes, qui permet une détection de groupes haptène,
(e) évaluation de la détection des groupes haptène sur le support, et
(f) corrélation des résultats de l'évaluation avec la qualité et/ou l'efficacité de la synthèse des récepteurs, les molécules de récepteur étant choisies parmi les acides nucléiques et/ou les analogues d'acide nucléique.

4. Procédé selon l'une des revendications 1 à 3, **caractérisé en ce que** l'on utilise un support microfluidique avec des canaux, de préférence avec des canaux fermés, dans lesquels se forment des zones prédéterminées avec les récepteurs immobilisés.

5. Procédé selon l'une des revendications 1 à 4, **caractérisé en ce que** l'on prépare un support avec plusieurs domaines de récepteurs différents, de préférence avec au moins 50 et de manière particulièrement préférée au moins 100 domaines de récepteurs différents.

6. Procédé selon l'une des revendications 1 à 5, **caractérisé en ce que** les groupes haptène sont choisis parmi les molécules organiques ayant un poids moléculaire allant jusqu'à 2000, qui sont reconnus par un partenaire de liaison spécifique via une interaction de haute affinité.

7. Procédé selon la revendication 6, **caractérisé en ce que** les groupes haptène sont choisis parmi la digoxine, la digoxigénine, le dinitrophénol et la biotine ou analogues de la biotine.

8. Procédé selon l'une des revendications 1 à 7, **caractérisé en ce que** l'on réalise une application en surface des groupes haptène sur le support.

9. Procédé selon l'une des revendications 1 à 8, **caractérisé en ce que** l'on réalise une application, spécifique d'un site, des groupes haptène sur le support.

10. Procédé selon l'une des revendications 1 à 9, **caractérisé en ce que** l'on applique les groupes haptène directement sur la surface du support.

11. Procédé selon l'une des revendications 1 à 10, **caractérisé en ce que** l'on introduit les groupes haptène dans des molécules espaceur, qui sont agencées entre la surface du support et les récepteurs.

12. Procédé selon l'une des revendications 1 à 11, **caractérisé en ce que** l'on introduit les groupes haptène en une ou plusieurs positions dans les récepteurs synthétisés sur le support.

13. Procédé selon l'une des revendications 1 à 12, **caractérisé en ce qu'**une application réversible des groupes haptène est effectuée.

14. Procédé selon l'une des revendications 1 à 12, **caractérisé en ce qu'**une application irréversible des groupes haptène est effectuée.

15. Utilisation de groupes haptène pour le contrôle de la synthèse de récepteurs sur un support, les récepteurs étant choisis parmi les acides nucléiques et/ou les analogues d'acide nucléique.
